(19) Europäisches Patentamt
European Patent Office
Office européen des brevets

(11) Publication number: 0 182 024 B1

(12) # EUROPEAN PATENT SPECIFICATION

(45) Date of publication of patent specification: 03.04.91

(51) Int. Cl.5: C07D 473/32, C07F 9/6561, A61K 31/52, A61K 31/665, A61K 31/675

(21) Application number: 85111354.8

(22) Date of filing: 09.09.85

The file contains technical information submitted after the application was filed and not included in this specification

(54) **Purine derivatives and their pharmaceutical use.**

(30) Priority: 20.09.84 GB 8423833
23.04.85 GB 8510331
16.08.85 GB 8520618

(43) Date of publication of application: 28.05.86 Bulletin 86/22

(45) Publication of the grant of the patent: 03.04.91 Bulletin 91/14

(84) Designated Contracting States: BE CH DE FR GB IT LI LU NL SE

(56) References cited:
EP-A- 0 108 285
EP-A- 0 141 927
EP-A- 0 152 316

(73) Proprietor: BEECHAM GROUP PLC
Beecham House Great West Road
Brentford Middlesex TW8 9BD(GB)

(72) Inventor: Harnden, Michael Raymond
47 Guildford Road
Horsham Sussex, RH12 1ND(GB)
Inventor: Jarvest, Richard Lewis
29 Beaconsfield Road
Surbiton Surrey(GB)

(74) Representative: Jones, Pauline et al
SmithKline Beecham, Corporate Patents,
Great Burgh, Yew Tree Bottom Road
Epsom, Surrey KT18 5XQ(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to compounds having antiviral activity, processes for their preparation and pharmaceutical compositions containing them.

EP-A-108285 (The Wellcome Foundation Limited) discloses purine derivatives having antiviral activity.

The compound 9-(4-hydroxy-3-hydroxymethylbut-1-yl) guanine of formula (A)

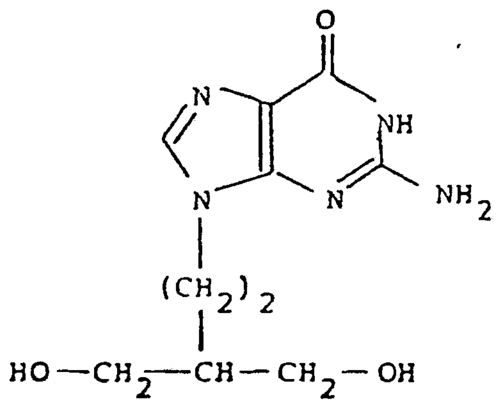

(A)

is disclosed in Synthetic Communications, 2(6), 345-351 (1972) but no pharmaceutical activity has been indicated for the compound in this document. We have subsequently shown that the compound of formula (A) does have pharmaceutical activity, and this is disclosed in EP-A-141927 (Beecham Group p.l.c.).

We have now prepared a series of analogues of the compound of formula (A) which has useful oral absorption properties and is converted in vivo to the compound of formula (A) which has anti-viral activity.

According to the present invention there is provided a compound of formula (I)

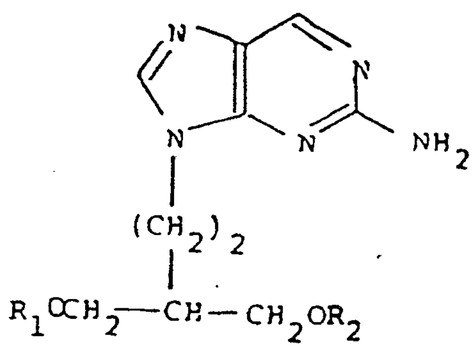

(I)

or a salt thereof, wherein $R_1$ and $R_2$ are each independently hydrogen, acyl or phosphate, provided that when one of $R_1$ or $R_2$ is phosphate, the other is hydrogen; or $R_1$ and $R_2$ are joined together to form a cyclic acetal group, a cyclic carbonate group or a cyclic phosphate group.

Acyl groups for $R_1$ and $R_2$ are those where the group $R_1O$- or $R_2O$- is a pharmaceutically acceptable carboxylic ester group which are

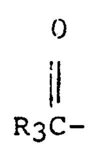

where $R_3$ is $C_{1-6}$ alkyl, $C_{1-6}$ alkoxy, or optionally substituted aryl.

As used herein the term 'aryl' includes phenyl which may be optionally substituted with one or two groups selected from $C_{1-6}$ alkyl, $C_{1-6}$ alkoxy or halo such as fluoro or chloro.

Preferably $R_3$ is methyl, ethyl, propyl, methoxy, or phenyl.

Suitably when $R_1$ and $R_2$ are joined together, they

$>C=O$, $>P(O)OH$ or $>C(C_{1-3}\ alkyl)_2$ such as $>C(CH_3)_2$.

constitute a group

A suitable example of a compound of formula (I) is the compound where one of $R_1$ or $R_2$ is

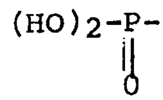

and the other is hydrogen.

In the case of compounds of formula (I) wherein one of $R_1$ or $R_2$ is an acyl or phosphate group, the compound exists in two enantiomeric forms. The invention includes both enantiomers in isolated form and mixtures thereof.

The compounds of the invention may be in crystalline form or as hydrates and it is intended that both forms are encompassed by the expression 'compound of formula (I)' used herein.

Salts of the compound of formula (I) are preferably pharmaceutically acceptable, but non-pharmaceutically acceptable salts are also within the scope of the present invention, since these are useful as intermediates in the preparation of pharmaceutically acceptable compounds.

Examples of pharmaceutically acceptable salts of the compound of formula (I) are acid addition salts formed with a pharmaceutically acceptable acid such as hydrochloric acid,orthophosphoric acid and sulphuric acid.

When the compound of formula (I) contains a phosphate group suitable salts include metal salts, such as aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)- amine.

Suitable compounds of formula (I) include;

2-amino-9-(4-hydroxy-3-hydroxymethylbut-1-yl)purine;
2-amino-9-(4-acetoxy-3-acetoxymethylbut-1-yl)purine;
2-amino-9-(4-acetoxy-3-hydroxymethylbut-1-yl)purine;
2-amino-9-(3-hydroxymethyl-4-methoxycarbonyloxybut -1-yl)purine;
2-amino-9-[2-(2,2-dimethyl-1,3-dioxan-5-yl)ethyl] purine;
2-amino-9-(4-propionyloxy-3-propionyloxymethylbut-1-yl) purine;
2-amino-9-(4-butyryloxy-3-hydroxymethylbut-1-yl)purine;
2-amino-9-(4-benzoyloxy-3-hydroxymethylbut-1-yl)purine;
2-amino-9-(4-hydroxy-3-hydroxymethylbut-1-yl)purine 4'-phosphate;
2-amino-9-(4-hydroxy-3-hydroxymethylbut-1-yl)purine 4': 4"phosphate;
and pharmaceutically aceptable salts thereof.

The compounds of the present invention are potentially useful in the treatment of infections caused by herpes viruses, such as herpes simplex type 1, herpes simplex type 2 and varicella zoster viruses.

Accordingly, the present invention also provides a compound of formula (I) or a pharmaceutically acceptable salt thereof, for use as an active therapeutic substance and in particular for use in the treatment of viral infections.

The compound of formula (I) wherein $R_1$ and $R_2$ are both hydrogen or a salt thereof may be prepared by hydrolysing the 1,3-dioxane ring of a compound of formula (II)

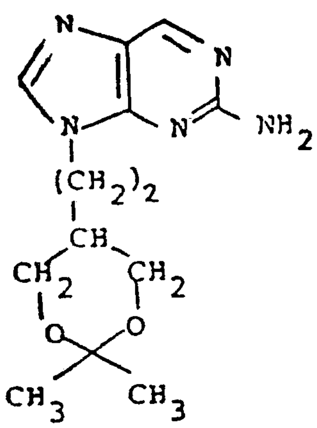

(II)

and subsequently, if necessary, converting the compound of formula (I) thus formed to the free base or to a different salt thereof.

Preferably the hydrolysis of the compound of formula (II) is carried out in acid medium, conveniently aqueous hydrochloric acid.

The compound of formula (II) is itself an example of a compound of formula (I) and may be prepared by reducing a compound of formula (III)

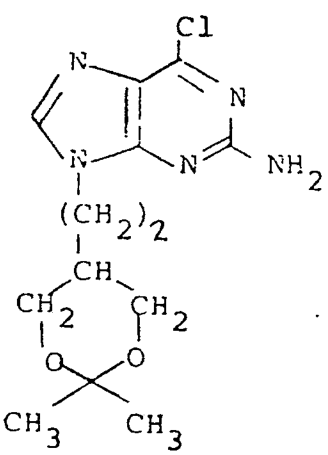

(III)

The reduction is preferably carried out catalytically, using palladium-on-charcoal, and the subsequent hydrolysis to the compound of formula (I) may be conveniently performed directly on the reaction product mixture.

The intermediate compound of formula (III) may be prepared by treating a compound of formula (IV)

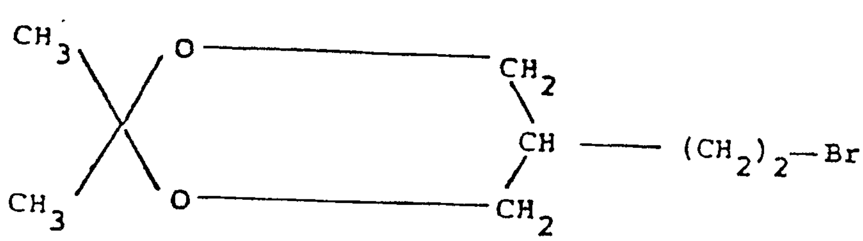

(IV)

with a compound of formula (V)

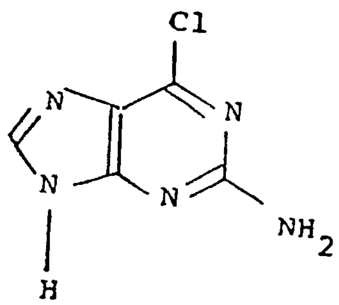

(V)

The reaction may be carried out in an inert organic solvent, preferably dimethylformamide, in the presence of an inorganic base, preferably potassium carbonate.

The compound of formula (IV) may itself be prepared by brominating a compound of formula (VI)

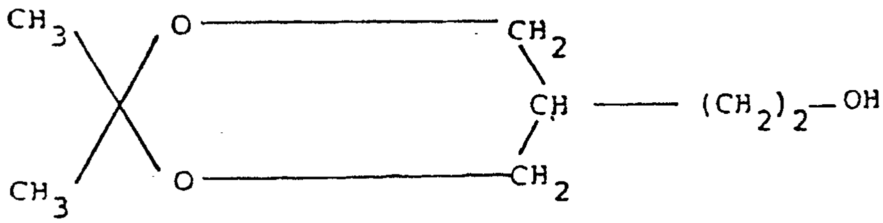

(VI)

The reaction is preferably carried out by treating the compound of formula (VI) with carbon tetrabromide and triphenylphosphine in an organic, aprotic solvent such as dimethylformamide.

The compound of formula (VI) may itself be prepared by treating a compound of formula (VII)

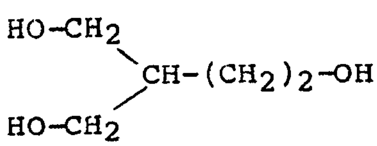

(VII)

with 2,2-dimethoxypropane and p-toluenesulphonic acid in the presence of acetone or tetrahydrofuran.

Compounds of formula (I) wherein $R_1$ and $R_2$ are acyl groups or are joined together to form a cyclic carbonate group can be prepared by reduction of a compound of formula (VIII)

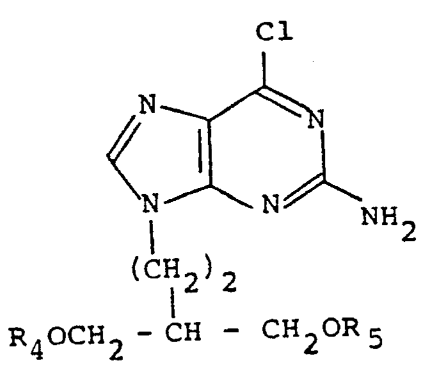

(VIII)

wherein $R_4$ and $R_5$ are the same or different acyl groups, or $R_4$ and $R_5$ are joined together to form a cyclic carbonate group.

Suitable acyl groups for $R_4$ and $R_5$ include groups

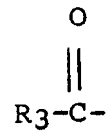

as hereinbefore defined.

The reduction is suitably carried out under conditions described above for the reduction of a compound of formula (III).

Compounds of formula (I) wherein $R_1$ and $R_2$ are acyl groups can be converted to a compound of formula (I) wherein $R_1$ and or $R_2$ are hydrogen by conventional deacylation or partial deacylation processes. For example, reaction with methanolic ammonia can be used to effect complete deacylation to yield compound of formula (I) wherein both $R_1$ and $R_2$ are hydrogen. Reaction with a mild base such as potassium carbonate can result in partial deacylation to produce a compound of formula (I) wherein one of $R_1$ or $R_2$ is hydrogen and the other is an acyl group.

Compounds of formula (VIII), may be prepared by treating the compound of formula (V) as hereinbefore defined, with a compound of formula (X)

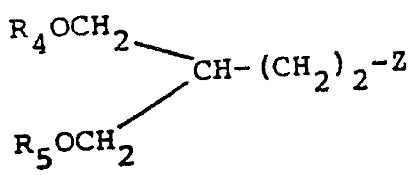

(X)

in which $R_4$ and $R_5$ are as defined in formula (VIII) and Z is a leaving group such as Cl, Br, or I, preferably Br.

The compound of formula (V) is a known compound.

Compounds of formula (X) in which Z is bromine may be prepared by brominating a compound of formula (XI).

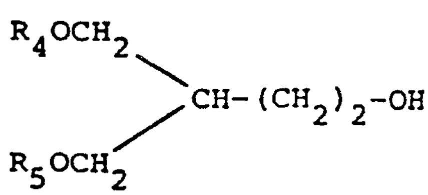

(XI)

preferably by treatment with carbon tetrabromide and triphenylphosphine in an organic, aprotic solvent, such as dimethylformamide.

Compounds of formula (X) in which Z is Cl or I may be prepared in an analogous manner.

Compounds of formula (XI) in which $R_4$ and $R_5$ are the same and are acyl groups may be prepared according to the following schematic process:

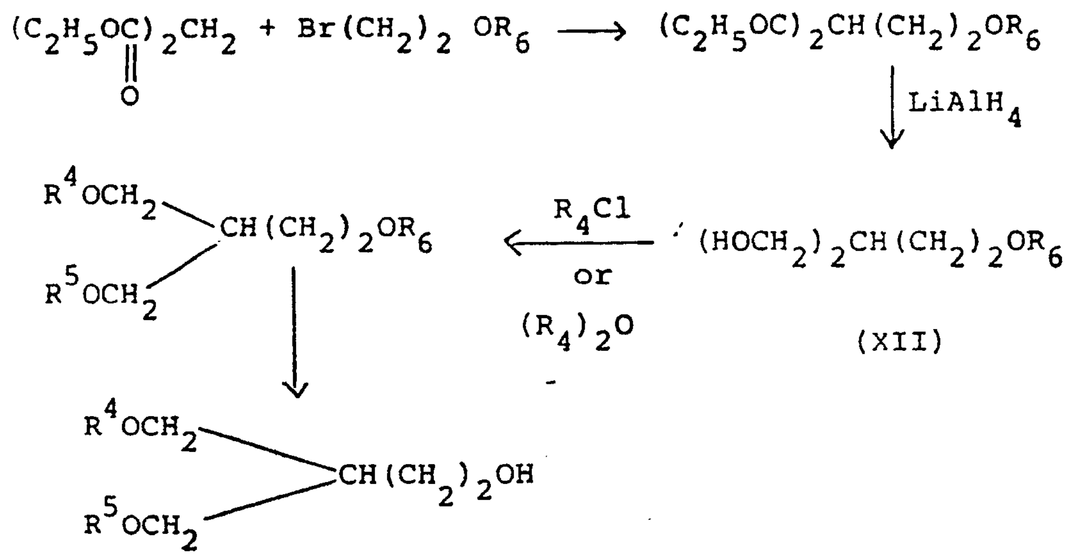


(XII)

(XI)

wherein $R^6$ is a removable protecting group.

Suitably $R_6$ is a group removable by hydrolysis or hydrogenolysis.

Preferably $R_6$ is a group removable by hydrogenolysis such as benzyl. This group can be removed by conventional methods for example by using hydrogen in the presence of a palladium/carbon catalyst.

Compounds of formula (XI) wherein $R_4$ and $R_5$ are joined together to form a cyclic carbonate group may be prepared by reaction of a compound formula (XII)

$$(HOCH_2)_2\ CH\ (CH_2)_2\ OR_6 \qquad (XII)$$

wherein $R_6$ is a hereinbefore defined with phosgene or 1,1' - carbonyldiimidazole, and thereafter if desired removing the protecting group $R_6$. The reaction is suitably carried out in a dry organic solvent such as pyridine at a temperature of from 0° - 50° c, conveniently at ambient temperature.

The above described processes for preparing the compound of formula (III) and compounds of formula (VIII) are also disclosed in Published European European Patent Application No. 0141 927.

Compounds of formula (I) wherein $R_1$ and/or $R_2$ is acyl may be prepared by esterifying a compound of formula (I) wherein $R_1$ and $R_2$ is hydrogen by conventional methods. If necessary during the esterification process the $-NH_2$ group and optionally also one of the $-OR_1$,or $-OR_2$ groups may be protected by a suitable protecting group such as trityl or monomethoxytrityl. The product is subsequently deprotected for example by treatment with acid such as acetic acid. For example, compounds of formula (I) wherein $R_1O$- and/or $R_2O$- is a carboxylic ester group may be prepared by reaction of a compound of formula (I) which has been optionally protected as described above with (a), an appropriate carboxylic acid chloride or (b) an appropriate carboxylic acid anhydride or (c) an appropriate carboxylic acid in the presence of a dehydrating agent such as dicyclohexylcarbodiimide (DCCI).

Compounds of formula (I) wherein $R_1$ and $R_2$ form a cyclic carbonate group can be prepared by reaction of a compound of formula (I) wherein $R_1$ and $R_2$ are hydrogen and the $NH_2$ group is optionally protected; with phosgene or 1,1-carbonyldiimidazole, and thereafter if necessary deprotecting the product. Suitable protecting groups for the $NH_2$ group include trityl and monomethoxytrityl as described above. The reaction is suitably carried out in a dry organic solvent such as pyridine at a temperature of from 0°-50° C, conveniently at ambient temperature.

Compounds of formula (I) wherein one of $R_1$ or $R_2$ is phosphate or $R_1$ and $R_2$ together form a cyclic phosphate can be prepared by treating a compound formula (XIII)

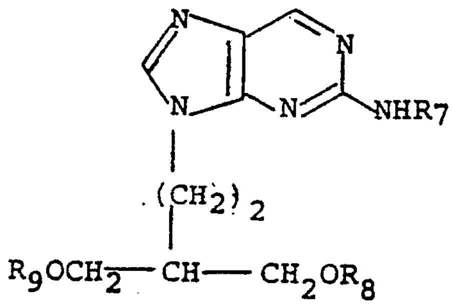

XIII

wherein $R_7$ is a protecting group and $R_8$ and $R_9$ are hydrogen or a protecting group provided that one of $R_8$ or $R_9$ is hydrogen; with a phosphorylating agent and thereafter if desired deprotecting resultant product. When $R_8$ and $R_9$ are both hydrogen, a cyclic phosphate compound is produced. Suitable protecting groups for $R_7$ and $R_8$ or $R_9$ are trityl or monomethoxytrityl. Deprotection of the resultant product can then be effected by treatment with acid such as acetic acid.

A suitable phosphorylating agent is phosphorus oxychloride, optionally in the presence of a base such as pyridine.

In addition, when one of $R_8$ or $R_9$ is a protecting group cyanoethyl phosphoric acid can be employed as a phosphorylating agent in order to produce a compound of formula (I) wherein one of $R_1$ or $R_2$ is phosphate.

The reaction product after treatment with cyanoethyl phosphoric acid is treated with aqueous ammonia, which yields the ammonium salt of the phosphate ester as the final product.

Compounds of formula (XIII) can be prepared by protection of a compound of formula (I) wherein $R_1$ and $R_2$ is hydrogen, for example by reaction with a trityl or monomethoxytrityl halide such as monomethoxytrityl chloride.

Alternatively compounds of formula (I) wherein $R_1$ and $R_2$ are joined together to form a cyclic phosphate can be prepared from a compound of formula (I) wherein one of $R_1$ or $R_2$ is phosphate and the other is hydrogen by cyclisation of the monophosphate for example using dicyclohexylcarbodiimide.

Compounds of formula (I) wherein one of $R_1$ or $R_2$ is acyl and the other is hydrogen or $R_1$ and $R_2$ together form a cyclic acetal can be prepared by reacting a compound of formula (I) wherein $R_1$ and $R_2$ are hydrogen with a compound of formula (XIV)

$$(R_{10})_m C(OR_{11})_n \qquad (XIV)$$

wherein
$R_{10}$ is $C_{1-6}$ alkyl,
$R_{11}$ is $C_{1-6}$ alkyl,
m is 0,1 or 2, and
n is an integer of 2, 3 or 4
provided that m + n is equal to 4,
and thereafter, if n is 3 or 4, hydrolysing the product.

When a compound of formula (I) in which $R_1$ and $R_2$ is a cyclic acetal is required, a compound of formula (XIV) wherein m is 2 and n is 2 is employed. For example, when m is 2, n is 2 and $R_{10}$ is methyl, the product is the compound of formula (II) as hereinbefore defined. The reaction is suitably carried out in an inert organic solvent such as tetrahydrofuran or N,N-dimethylformamide, in the presence of an acid such as p-toluene sulphonic acid.

Where necessary the subsequent hydrolysis step is an aqueous hydrolysis preferably carried out in the presence of an acid such as p-toluene sulphonic acid.

Compounds of formula (XIV) are known compounds or can be prepared from known compounds by known methods.

Compounds of formula (I) or pharmaceutically acceptable salts thereof may be formulated for use in a pharmaceutical composition. Accordingly, in a further aspect of the invention, there is provided a pharmaceutical composition which comprises a compound of formula (I) or pharmaceutically acceptable salt thereof together with a pharmaceutically acceptable carrier or excipient.

A composition which may be administered by the oral route to humans may be compounded in the form of a syrup, tablet or capsule. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the composition may be in the form of a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art, or an ointment composition.

Preferably, the composition of this invention is in unit dosage form or in some other form that the patient may administer to himself a single dose. A suitable dosage unit might contain from 50 mg to 1 g of active ingredient, for example 100 to 500 mg.

Such doses may be administered 1 to 4 times a day or more usually 2 or 3 times a day. The effective dose of compound will in general be in the range of from 1.0 to 20 mg/kg of body weight per day or more usually 2.0 to 10 mg/kg per day.

No toxicological effects are indicated at the above described dosage levels.

In a further aspect of the invention there is provided the use of a compound of formula (I) or a pharmaceutically acceptable salt thereof, in the manufacture of a medicament for use in the treatment of viral infections.

The following examples illustrate the invention.

Example 1

2-Amino-9-(4-hydroxy-3-hydroxymethylbut-1-yl)purine

Method A

To a solution of 2-amino-6-chloro-9-[2-(2,2-dimethyl-1, 3-dioxan-5-yl)ethyl]purine (0.54g, 1.75mmol) in ethanol (10ml) and cyclohexene (20ml) was added 10% palladium-on-charcoal (400mg) and the solution was refluxed for 7 hours. A further quantity of catalyst (200mg) was added and the solution was refluxed overnight. The solution was filtered and washed through with methanol. To the filtrate was added hydrochloric acid (5M , 0.3ml) and water (0.7ml) and the solution was stirred for 30 minutes at room temperature. The solution was neutralised by addition of aqueous sodium bicarbonate and the solvent was removed. The residue was purified by column chromatography on silica gel eluting with chloroform-methanol (5:1, 4:1) to afford 2-amino-9-(4-hydroxy-3-hydroxymethylbut-1-yl)purine as a crystalline solid (150mg, 36%), m.p. 156-158°C; $\lambda$max ($H_2O$) 242 and 303 nm; $\nu$max (KBr) 3320, 3210, 1640, 1610, 1580, and 1430 $cm^{-1}$; $\delta_H$ [$(CD_3)_2SO$] 1.47 (1H, m, 3'-H), 1.78 (2H, q, J 7.2Hz, 2'-H), 3.3-3.5 (4H, m, 2 x 4'-H), 4.12 (2H, t, J 7.4Hz, 1'-H), 4.42 (2H, t, J 5.2Hz, $D_2O$ exchangeable, 2 x OH), 6.45 (2H, s, $D_2O$ exchangeable, 2-$NH_2$), 8.06 (1H, s, 8-H), and 8.56 (1H, s, 6-H); (Found: C, 50.61; H, 6.45; N, 29.62 %. $C_{10}H_{15}N_5O_2$ requires: C, 50.62; H, 6.37; N, 29.52 %).

Method B (alternative reduction reaction)

To a solution of ammonium formate in methanol (400mM , 3ml) were added 2-amino-6-chloro-9-[2-(2,2-dimethyl-1,3-dioxan-5-yl)ethyl]purine (90mg, 0.3mmol) and 10% palladium-on-charcoal (28mg) and the mixture was heated under reflux. After 1.5 hours reduction to 2-amino-9-[2-(2,2-dimethyl-1,3-dioxan-5-yl)-ethyl]purine was complete.

Example 2

9-(4-Acetoxy-3-acetoxymethylbut-1-yl)-2-aminopurine

A suspension of 9-(4-acetoxy-3-acetoxymethylbut-1-yl)-2-amino-6-chloropurine (0.36g, 1.0mmol) and 10% palladium-on-charcoal (30mg) in methanol containing ammonium formate (400mM , 10ml) was heated under reflux for 30 minutes. The mixture was allowed to cool, filtered and the solvent removed. The residue was taken up in water and the solution extracted twice with chloroform. The organic layers were combined, dried (magnesium sulphate) and the solvent removed to afford 9-(4-acetoxy-3-acetoxymethylbut-1-yl)-2-aminopurine (0.29g, 90%). Recrystallisation from ethyl acetate-hexane gave white shiny plates (0.25g, 78%) m.p. 102-104° C; $\lambda$max (MeOH) 222 (27,500), 244 (4,890), and 309 (7,160)nm; $\nu$max (KBr) 3340, 3170, 1745, 1730, 1660, 1615 and 1580cm$^{-1}$; $\delta_H$ ($CDCl_3$) 1.90-2.05 (3H, m, 2'-H and 3'-H), 2.07 (6H, s, 2 x $CH_3$), 4.15 (4H, d, J 5.2 Hz, 2x4'-H), 4.21 (2H, t, J 7.2Hz, 1'-H), 5.16 (2H, br s, 2-$NH_2$), 7.79 (1H, s, 8-H), and 8.70 (1H, s, 6-H); (Found: C, 52.10; H, 6.00; N, 21.49%. $C_{14}H_{19}N_5O_4$ requires C, 52.33; H, 5.96; N, 21.79%).

Example 3

2-Amino-9-(4-hydroxy-3-hydroxymethylbut-1-yl)purine

To a suspension of 9-(4-acetoxy-3-acetoxymethylbut-1-yl)-2-amino-6-chloropurine (4.86g, 13.7mmol) in methanol (140ml) containing ammonium formate (400mM ) was added 10% palladium-on-charcoal (0.4g) and the mixture was heated under reflux for 40 minutes. After cooling the solution was filtered and the solvent removed. The residue was taken up in water and extracted with chloroform (100ml and 50ml). The organic layers were combined, dried (magnesium sulphate) and the solvent removed. The residue was dissolved in methanol saturated with ammonia at 0° C (150ml) and the solution was stirred for 20 hours. The solvent was removed and the residue suspended in chloroform (20ml) and filtered. The solid was recrystallised from isopropanol-water and a second recrystallisation was carried out from the mother liquors from ethanol (total 2.71g, 83%).

Example 4

9-(4-Acetoxy-3-hydroxymethylbut-1-yl)-2-aminopurine

To a solution of 9-(4-acetoxy-3-acetoxymethylbut-1-yl)-2-aminopurine (0.48g, 1.5mmol) in methanol (9ml) was added anhydrous potassium carbonate (14mg, 0.1mmol) and the solution was stirred for 20 minutes. Two drops of glacial acetic acid were added, the solution was filtered and the solvent was removed. The residue was purified by column chromatography on silica gel eluting with chloroform-methanol (15:1, 10:1) to afford 9-(4-acetoxy-3-hydroxymethyl-1-yl)-2-aminopurine as a white crystalline solid (124mg, 30%), m.p. 166-168° ; $\nu_{max}$ (KBr) 3440, 3220, 1720, 1650, 1615, and 1580cm$^{-1}$; $\delta_H$ [$(CD_3)_2SO$] 1.68 (1H, m, 3'-H), 1.82 (2H, m, 2'-H), 1.98 (3H, s, $CH_3$), 3.41 (2H, t, J4.8Hz, $D_2O$ exchange gives d, $CH_2OH$), 3.9 - 4.05 (2H, AB part of ABX, $J_{AB}$ 10.9Hz and $J_{AX}$ = $J_{BX}$ 5.8Hz, $CH_2OCO$), 4.12 (2H, t, J 7.2Hz, 1'-H), 4.62 (1H, t, J 5.0Hz, $D_2O$ exchangeable, OH), 6.44 (2H, s, $D_2O$ exchangeable, 2-$NH_2$), 8.07 (1H, s, 8-H), and 8.56 (1H, s, 6-H); (Observed M$^+$, 279.1326. $C_{12}H_{17}N_5O_3$ requires 279.1331).

Example 5

2-Amino-9-(3-hydroxymethyl-4-methoxycarbonyloxybut-1-yl)purine

To a suspension of 2-amino-9-(4-hydroxy-3-hydroxymethylbut-1-yl)purine (237mg, 1.0mmol) in dry tetrahydrofuran (3ml) were added p -toluenesulphonic acid monohydrate (0.21g, 1.1mmol) and tetramethyl orthocarbonate (0.53ml, 4.0mmol) and the mixture was stirred for 100 minutes. Water (0.8ml) was added and after a further 15 minutes the solution was neutralised by addition of aqueous sodium bicarbonate. The solvent was removed and the residue was extracted with chloroform-methanol (3:1). The solvent was removed and the residue was purified by column chromatography on silica gel eluting with chloroform-methanol (10:1) to afford 2-amino-9-(3-hydroxymethyl-4-methoxycarbonyloxybut-1-yl)purine which was obtained as a white crystalline solid after trituration with ethyl acetate (65mg, 22%), m.p. 129 - 132°; $\nu_{max}$ - (KBr) 3440, 3220, 1745, 1650, 1615, and 1580cm$^{-1}$ $\delta_H$ [$(CD_3)_2SO$] 1.73 (1H, m, 3'-H), 1.81 (2H, m, 2'-H), 3.41 (2H, t, J 5.1Hz, $D_2O$ exchange gives d, $CH_2OH$) 3.68 (3H, s, $CH_3$), 4.0 - 4.2 (4H, m, $CH_2OCO$ and 1'-H), 4.65 (1H, t, J 5.2Hz, $D_2O$ exchangeable, OH), 6.44 (2H, s, $D_2O$ exchangeable, 2-$NH_2$), 8.06 (1H, s, 8-H), and 8.55 (1H, s, 6-H); (Observed $M^+$, 295.1286, $C_{12}H_{17}N_5O_4$ requires 295.1280).

Example 6

2-Amino-9-[2-(2,2-dimethyl-1,3-dioxan-5-yl)ethyl]purine

To a suspension of 2-amino-9-(4-hydroxy-3-hydroxymethylbut-1-yl)purine (240mg, 1.0mmol) in N,N-dimethylformamide (3ml) were added p -toluenesulphonic acid monohydrate (210mg,1.1mmol) and 2,2-dimethoxypropane (0.62ml, 5.0mmol) and the solution was stirred for 30 minutes. Potassium carbonate (110mg, 0.8mmol) was added and the solution was stirred for a further 30 minutes. Water (10ml) was added and the solution was extracted with chloroform (3 x 8ml). The organic layers were combined, dried (magnesium sulphate) and the solvent removed. Trituration with toluene-ether afforded 2-amino-9-[2-(2,2-dimethyl-1,3-dioxan-5-yl)ethyl]purine as a white crystalline solid (262mg, 94%) which was recrystallised from ethyl acetate-hexane (216mg, 78%), m.p. 118 - 120°; $\lambda_{max}$ (MeOH) 221 (27,200), 244 (4,920), and 308 (7,130)nm; $\nu_{max}$ (KBr) 3450, 3140, 1635, 1615, 1580, and 1435cm$^{-1}$; $\delta_H$ [$(CD_3)_2SO$] 1.26 (3H, s, $CH_3$), 1.33 (3H, s, $CH_3$), 1.58 (1H, m, 3'-H), 1.74 (2H, q, J 7.1Hz, 2'-H), 3.54 (2H, dd, J 11.8Hz and 8.5Hz, 2 x $H_{ax}$), 3.78 (2H, dd, J 11.8Hz and 4.4Hz, 2 x $H_{eq}$), 4.07 (2H, t, J 7.2Hz, 1'-H), 6.46 (2H, s, $D_2O$ exchangeable, 2-$NH_2$), 8.09 (1H, s, 8-H), and 8.56 (1H, s, 6-H; (Found: C, 56.09; H, 6.91; N, 24.88%. $C_{13}H_{19}N_5O_2$ requires C, 56.30; H, 6.91; N, 25.25%).

Example 7

2-Amino-9-(4-propionyloxy-3-propionyloxymethylbut-1-yl)purine

A solution of 2-amino-9-(4-hydroxy-3-hydroxymethylbut-1-yl)purine (0.21g, 0.9mmol), 4-dimethylaminopyridine (10mg) and propionic anhydride (0.64ml, 5.0mmol) in N,N-dimethylformamide (5ml) was stirred for 16 hours. The solvent was removed and the residue was partitioned between aqueous sodium bicarbonate and chloroform. The organic layer was dried (magnesium sulphate) and the solvent was removed. The residue was purified by column chromatography on silica gel eluting with chloroform-methanol (20:1) to give 2-amino-9-(4-propionyloxy-3-propionyloxymethylbut-1-yl)-purine (160mg, 51%) which was recrystallised from ethyl acetate-hexane (115mg, 37%), m.p. 77.5 - 79°; $\lambda_{max}$ (EtOH) 222 (27,300), 244 (5,020), and 309 (7,110)nm; $\nu_{max}$ (KBr) 3390, 3210, 1735, 1650, 1605, 1580, 1525, 1475, and 1425cm$^{-1}$; $\delta_H$ ($CDCl_3$) 1.14 (6H, t, J 7.6Hz, 2 x $CH_3$), 1.96 (3H, m, 2'-H and 3'-H), 2.34 (4H, q, J 7.6Hz, 2 x $CH_2CH_3$), 4.15 (4H, d, J 5.5Hz, 2 x $CH_2OOC$), 4.21 (2H, t, J 7.0Hz, 1'-H), 5.05 (2H, s, $D_2O$ exchangeable, 2-$NH_2$), 7.77 (1H, s, 8-H), and 8.69 (1H, s, 6-H); (Observed $M^+$ 349.1752. $C_{16}H_{23}N_5O_4$ requires 349.1751).

9-(3-Hydroxymethyl-4-monomethoxytrityloxybut-1-yl)-2-monomethoxy tritylaminopurine (Example 8) and

9-(4-Hydroxy-3-hydroxymethylbut-1-yl)-2-monomethoxytritylamino purine (Example 9)

To a suspension of 2-amino-9-(4-hydroxy-3-hydroxymethylbut-1-yl)purine (2.37g, 10mmol) in N,N-dimethylformamide (40ml) containing 4-dimethylaminopyridine (30mg) and triethylamine (4.2ml) was added a solution of monomethoxytrityl chloride (6.8g, 22mmol) in N,N-dimethylformamide (60ml) over a period of 40 minutes. The solution was stirred for a further 40 minutes, methanol (1ml) was added and the solvent was removed. The residue was taken up in chloroform and washed with water and dilute aqueous sodium bicarbonate. The organic layer was dried (magnesium sulphate) and the solvent was removed. The residue was purified by column chromatography on silica gel eluting with chloroform-methanol mixtures (40:1 to 6:1).

The first product to elute was 9-(3-hydroxymethyl-4-monomethoxytrityloxybut-1-yl)-2-monomethoxytritylaminopurine which was further purified by a second silica gel column eluting with chloroform-methanol (40:1) and obtained as a colourless foam (3.34g, 43%); $\lambda_{max}$ (EtOH) 227 (47,400) and 312 (6,450)-nm; $\nu_{max}$ (KBr) 3430, 1615, 1580, 1510, 1490, and 1415cm$^{-1}$; $\delta_H$ [$(CD_3)_2SO$] 1.37 (2H, m, 2'-H), 1.49 (1H, m, 3'-H), 2.8 - 2.9 (2H, m, $CH_2OC$), 3.2 - 3.4 (2H, m, $\underline{CH}_2OH$), 3.64 (5H, m, 1'-H and $OCH_3$), 3.73 (3H, s, $OCH_3$), 4.40 (1H, t, J 5.0Hz, $D_2O$ exchangeable, $\underline{OH}$), 6.7-7.4 (28H, m, Ar-H), 7.46 (1H, s, $D_2O$ exchangeable, 2-NH), 7.88 (1H, s, 8-H), and 8.53 (1H, s, 6-H): (Found: C, 77.28; H, 6.27; N,8.94%. $C_{50}H_{47}N_5O_4$ requires C, 76.80; H,6.06; N,8.96%).

The second product to elute was 9-(4-hydroxy-3-hydroxymethylbut-1-yl)-2-monomethoxytritylaminopurine which was obtained as a white crystalline solid after trituration and filtration from ether (2.07g, 41%), m.p. 181 - 183°; $\lambda_{max}$ (EtOH) 227 (36,000) and 312 (6,780)nm; $\nu_{max}$ (KBr) 3390, 1615, 1580, 1525, 1510, 1490, and 1420cm$^{-1}$; $\delta_H$ [$(CD_3)_2SO$] 1.30 (1H, m, 3'-H), 1.39 (2H, q, J 6.8Hz, 2'-H), 3.15 - 3.35 (4H, m, 2 x 4'-H), 3.70 (3H, s, $OCH_3$), 3.76 (2H, t, J 7.2Hz, 1'-H), 4.33 (2H, t, J 5.1Hz, $D_2O$ exchangeable, 2 x OH), 6.8 - 7.4 (14H, m, Ar-H), 7.52 (1H, s, $D_2O$ exchangeable, 2-NH), 7.97 (1H, s, 8-H), and 8.52 (1H, s, 6-H); (Found: C, 70.49; H, 6.24; N, 13.41%. $C_{30}H_{31}N_5O_3$ requires C, 70.71; H, 6.13; N, 13.74%).

Example 10

2-Amino-9-(4-butyryloxy-3-hydroxymethylbut-1-yl)purine

To a solution of 9-(3-hydroxymethyl-4-monomethoxytrityloxybut-1-yl)-2-monomethoxytritylaminopurine (0.70g, 0.9mmol) and 4-dimethylaminopyridine (10mg) in N,N-dimethylformamide (5ml) was added butyric anhydride (0.29ml, 1.8mmol) and the solution was stirred for 15 minutes. Methanol (1ml) was added and the solvent was removed. The residue was taken up in 80% acetic acid (9ml) and the solution was stirred at 70° for 30 minutes. Water (2ml) was added and the solution was extracted with hexane (2 x 10ml). The aqueous layer was retained and the solvent was removed. The residue was partitioned between saturated aqueous sodium bicarbonate and chloroform and the organic layer was dried (magnesium sulphate) and the solvent removed. The residue was purified by column chromatography on silica gel eluting with chloroform-methanol (16:1) to afford 2-amino-9-(4-butyryloxy-3-hydroxymethylbut-1-yl)purine which was obtained as a white crystalline solid after trituration with methanol (188mg, 68%), m.p. 125 - 127°; $\lambda_{max}$ (MeOH) 222 (27,600), 243 (4,830), and 308 (6,950)nm; $\nu_{max}$ (KBr) 3190, 1730, 1640, 1620, and 1580cm$^{-1}$; $\delta_H$ [$(CD_3)_2SO$] 0.85 (3H, t, J 7.4Hz, $CH_3$), 1.50 (2H, sextet, J 7.3Hz, $CH_2\underline{CH}_2CH_3$), 1.68 (1H, m, 3'-H), 1.82 (2H, m, 2'-H), 2.23 (2H, t, J 7.4Hz, $\underline{CH}_2CH_2CH_3$), 3.42 (2H, t, J 5.2Hz, $\overline{D_2O}$ exchange gives d, $\underline{CH}_2OH$), 3.95 - 4.1 (2H, ABX, $J_{AB}$ 11.0Hz, $J_{AX}$ = $J_{BX}$ 5.8Hz, $CH_2OOC$), 4.12 (2H, t, J 7.3Hz, 1'-H), 4.62 (1H, t, J 4.9Hz, $D_2O$ exchangeable, OH), 6.44 (2H, s, $D_2O$ exchangeable, 2-$NH_2$), 8.06 (1H, s, 8-H), and 8.56 (1H, s, 6-H); (Found: C, 54.41; H, 6.91; N, 22.70%. $C_{14}H_{21}N_5O_3$ requires C, 54.71; H, 6.89; N, 22.79%).

Example 11

2-Amino-9-(4-benzoyloxy-3-hydroxymethylbut-1-yl)purine

To a solution of 9-(3-hydroxymethyl-4-monomethoxytrityloxybut-1-yl)-2-monomethoxytritylaminopurine (0.70g, 0.9mmol) and 4-dimethylaminopyridine (10mg) in N,N-dimethylformamide (5ml) was added benzoic anhydride (0.61g, 2.7mmol) and the solution was stirred for 1 hour. Methanol (1ml) was added and the solvent was removed. The residue was taken up in 80% acetic acid (9ml) and the solution was stirred at

80° for 20 minutes. Water (3ml) was added and the solution was extracted with hexane (2 x 10ml). The aqueous layer was retained and the solvent was removed. The residue was partitioned between saturated aqueous sodium bicarbonate and chloroform and the organic layer was dried (magnesium sulphate) and the solvent removed. The residue was purified by column chromatography on silica gel eluting with chloroform-methanol (14:1) to afford 2-amino-9-(4-benzoyloxy-3-hydroxymethylbut-1-yl)purine which was obtained as a white crystalline solid after trituration with methanol (235mg, 76%), m.p. 116-118°; $\lambda_{max}$ (MeOH) 223 (36,700) and 309 (6,680)nm; $\nu_{max}$ (KBr) 3320, 1710, 1610, and 1580cm$^{-1}$; $\delta_H$ [$(CD_3)_2SO$] 1.83 (1H, m, 3'-H), 1.93 (2H, q, J 7.1Hz, 2'-H), 3.52 (2H, t, J 5.3Hz, $D_2O$ exchange gives d, $CH_2OH$), 4.19 (2H, t, J 7.0Hz, 1'-H), 4.2 - 4.3 (2H, ABX, $J_{AB}$ 11.0Hz, $J_{AX}$ = $J_{BX}$ 5.6Hz, $CH_2OOC$), 4.69 (1H, t, J 5.2Hz, $D_2O$ exchangeable, OH), 6.43 (2H, s, $D_2O$ exchangeable, 2-$NH_2$), 7.5 - 7.9 (5H, m, $C_6H_5$), 8.10 (1H, s, 8-H), and 8.55 (1H, s, 6-H); (Found: C, 59.20; H, 5.63; N, 20.82%. $C_{17}H_{19}N_5O_3$ requires C, 59.81; H, 5.61; N, 20.52%).

## Example 12

### 2-Amino-9-(4-hydroxy-3-hydroxymethylbut-1-yl)purine 4'-phosphate

To an ice-cooled solution of phosphorus oxychloride (0.10ml, 1.1mmol) in pyridine (2ml) was added dropwise over 15 minutes a solution of 9-(3-hydroxymethyl-4-monomethoxytrityloxybut-1-yl)-2-monomethoxytritylaminopurine (0.78g, 1.0mmol) in pyridine (2ml). The solution was stirred for a further 5 minutes at 0° and then for 30 minutes at room temperature. The solution was added dropwise to a solution of sodium bicarbonate (0.5g, 6.0mmol) in water (7ml). The solvent was removed and the residue was taken up in 80% acetic acid (10ml) and the solution was stirred at 70° for 25 minutes. The solvent was removed and the residue was taken up in water and brought to pH 6 by addition of ammonia. The solution was extracted twice with chloroform and the solvent was removed. The residue was purified by preparative high pressure liquid chromatography on a $C_{18}$ reverse-phase µ-Bondapack column eluting with 3% methanol in ammonium acetate buffer (pH 4.5, 50mM ) to afford 2-amino-9-(4-hydroxy-3-hydroxymethylbut-1-yl)purine 4'-phosphate as a hygroscopic white powder (85mg, 25%); $\lambda_{max}$ ($H_2O$) 220, 241, and 303nm; $\nu_{max}$ (KBr) 3410, 1660, 1620, and 1580cm$^{-1}$; $\delta_H$ [$(CD_3)_2SO$] 1.57 (1H, m, 3'-H), 1.77 (2H, m, 2'-H), 3.37 (2H, d, J 4.4Hz, $CH_2OH$), 3.77 (2H, t, J 5.6Hz, $CH_2OP$), 4.12 (2H, t, J 7.4Hz, 1'-H), 6.48 (2H, s, $D_2O$ exchangeable, 2-$NH_2$), 8.08 (1H, s, 8-H), and 8.54 (1H, s, 6-H); (Found: C, 35.53; H, 5.93; N, 22.24%. $C_{10}H_{16}N_5O_5P$ . $0.5NH_3$ . $H_2O$ requires C, 34.94; H, 5.72; N, 22.41%). $C_{10}H_{16}N_5O_5P$ . $0.5NH_3$ . $H_2O$ requires C, 34.94; H, 5.72; N, 22.41%).

## Example 13

### 2-Amino-9-(4-hydroxy-3-hydroxymethylbut-1-yl)purine 4':4"-phosphate

To an ice-cooled solution of phosphorus oxychloride (93µl, 1.0mmol) in pyridine (2ml) was added dropwise over 45 minutes a solution of 9-(4-hydroxy-3-hydroxymethylbut-1-yl)-2-monomethoxytritylaminopurine (0.46g, 0.9mmol) in pyridine (4ml). The solution was stirred for a further 20 minutes at room temperature and was then added dropwise to a solution of sodium bicarbonate (0.34g, 4.0mmol) in water (6ml). The solvent was removed and the residue was taken up in 80% acetic acid (9ml) and the solution was stirred at 70° for 25 minutes. The solvent was removed and the residue was taken up in water and brought to pH 6 by addition of ammonia. The solution was extracted twice with chloroform and the solvent was removed. The residue was purified by preparative high pressure liquid chromatography on a $C_{18}$ reverse-phase µ-Bondapack column eluting with 4% methanol in ammonium acetate buffer (pH 4.5, 50mM ) to afford 2-amino-9-(4-hydroxy-3-hydroxymethylbut-1-yl) purine 4':4" -phosphate as a white powder (225mg, 75%); $\lambda_{max}$ ($H_2O$) 220, 242, and 303nm; $\nu_{max}$(KBr) 2900 - 3200 (br), 1705, 1615, and 1580cm$^{-1}$; $\delta_H$ [$(CD_3)_2SO$] 1.63 (1H, m, 3'-H), 1.74 (2H, q, J 7.0Hz, 2'-H), 3.80 (2H, q, J 9.2Hz, 2 x $H_{ax}$), 3.98 (2H, ddd, J 14.3, 10.9, and 3.5Hz, 2 x $H_{eq}$), 4.08 (2H, t, J 7.1Hz, 1'-H), 6.51 (2H, s, $D_2O$ exchangeable, 2-$NH_2$), 8.10 (1H, s, 8-H), and 8.56 (1H, s, 6-H); (Found: C, 36.41; H, 5.18; N, 22.38%. $C_{10}H_{14}N_5O_4P$. 0.3 $NH_3$. $1.5H_2O$ requires C, 36.25; H, 5.45; N, 22.40%).

BIOLOGICAL DATA

Xanthine Oxidase Catalysed Oxidation of 2-Amino-9-(4-hydroxy-3-hydroxymethylbut-1-yl)purine

To an aqueous solution of 2-amino-9-(4-hydroxy-3-hydroxymethylbut-1-yl)purine (0.5mM, 0.7ml; pH7) was added bovine milk xanthine oxidase (20µl, 0.4 unit). Dissolved atmosphere oxygen was allowed to act as electron acceptor and changes in the UV spectrum were measured. After 4 minutes 25% conversion had occurred and after 2.5 hours conversion was essentially complete. The oxidation product was identified as 9-(4-hydroxy-3-hydroxymethylbut-1-yl)guanine by its UV spectrum and HPLC retention time.

(Incubation of 9-(4-hydroxy-3-hydroxymethylbut-1-yl)guanine with xanthine oxidase under identical conditions resulted in no change over a 2 hour period.)

Oral Absorption of 2-Amino-9-(4-hydroxy-3-hydroxymethylbut-1-yl) purine and 2-Amino-9-(4-acetoxy-3-acetoxymethylbut-1-yl)purine and their Conversion to 9-(4-hydroxy-3-hydroxymethylbut-1-yl) guanine in Mice

Procedure

2-Amino-9-(4-hydroxy-3-hydroxymethylbut-1-yl)purine, 2-amino-9-(4-acetoxy-3-acetoxymethylbut-1-yl)-purine and 9-(4-hydroxy-3-hydroxymethylbut-1-yl)guanine were administered by oral gavage (0.2mmoles/kg in 0.1ml of 1% carboxymethyl cellulose) to 20g female Balb/C mice which had been starved for 18 hours. Fifteen 60 and 180 minutes later, blood was collected from three mice per time point by cardiac puncture using heparinised syringes. Equal aliquots at each time were pooled and an equal volume of 16% trichloroacetic acid added. Following centrifugation (8,500g) to remove precipitated proteins, 0.5ml of supernatant was immediately added to 0.1ml of saturated sodium bicarbonate solution and the resulting mixture analysed by high performance liquid chromatography or stored at -20°C prior to analysis.

Results

| | Administered Compound | Concentration of 9-(4-hydroxy-3-hydroxymethylbut-1-yl)guanine (μg/ml) in blood at stated times after administration 15 min | 1 hr | 3 hr |
|---|---|---|---|---|
| Expt. 1 | 9-(4-Hydroxy-3-hydroxymethylbut-1-yl)guanine | 0.7 | 0.4 | 0.1 |
| Expt. 1 | 2-Amino-9-(4-hydroxy-3-hydroxymethylbut-1-yl)purine | 3.0 | 2.2 | 0.3 |
| Expt. 2 | 9-(4-Hydroxy-3-hydroxymethylbut-1-yl)guanine | 1.3 | 1.0 | 0.4 |
| Expt. 2 | 2-Amino-9-(4-hydroxy-3-hydroxymethylbut-1-yl)purine | 4.6 | 2.8 | 0.6 |
| Expt. 2 | 2-Amino-9-(4-acetoxy-3-acetoxymethylbut-1-yl)purine | 18.7 | 4.3 | 0.3 |

| | Administered Compound | Concentration of 9-(4-hydroxy-3-hydroxymethylbut-1-yl)guanine (μg/ml) in blood at stated times after administration | | |
|---|---|---|---|---|
| | | 15 min | 1 hr | 3 hr |
| Expt. 3 | 9-(4-Hydroxy-3-hydroxymethylbut-1-yl)guanine | 1.4 | 1.1 | 0.5 |
| | 2-Amino-9-(4-hydroxy-3-hydroxymethylbut-1-yl)purine | 4.8 | 4.6 | 1.2 |
| | 9-(4-acetoxy-3-hydroxymethylbut-1-yl)-2-aminopurine | 12.9 | 5.1 | 0.3 |
| | 2-Amino-9-(3-hydroxymethyl-4-methoxycarbonyloxybut-1-yl)purine | 13.7 | 5.6 | 0.7 |
| | 2-Amino-9-[2-(2,2-dimethyl-1,3-dioxan-5-yl)ethyl]purine | 8.4 | 2.8 | 0.8 |
| Expt. 4 | 9-(4-Hydroxy-3-hydroxymethylbut-1-yl)guanine | 1.1 | 0.9 | 0.4 |
| | 2-Amino-9-(4-hydroxy-3-hydroxymethylbut-1-yl)purine | 3.5 | 4.0 | 0.8 |
| | 2-Amino-9-(4-propionyloxy-3-propionyloxymethylbut-1-yl)purine | 20.0 | 6.6 | 0.5 |
| | 2-Amino-9-(4-butyryloxy-3-hydroxymethylbut-1-yl)purine | 16.2 | 7.1 | 0.5 |
| | 2-Amino-9-(4-benzoyloxy-3-hydroxymethylbut-1-yl)purine | 16.0 | 6.6 | 0.3 |

| | Administered Compound | Concentration of 9-(4-hydroxy-3-hydroxymethylbut-1-yl)guanine (μg/ml) in blood at stated times after administration | | |
|---|---|---|---|---|
| | | 15min | 1hr | 3hr |
| Expt.5 | 9-(4-hydroxy-3-hydroxymethylbut-1-yl)guanine | 1.3 | 1.0 | 0.2 |
| | 2-amino-9-(4-hydroxy-3-hydroxymethylbut-1-yl)purine | 4.1 | 4.1 | 1.4 |
| | 2-amino-9-(4-hydroxy-3-hydroxymethylbut-1-yl)purine 4′-phosphate | 2.2 | 4.3 | 1.3 |
| | 2-amino-9-(4-hydroxy-3-hydroxymethylbut-1-yl) purine 4′:4″-phosphate | 0.2 | 0.2 | 0.7 |

## Claims

1. A compound of formula (I)

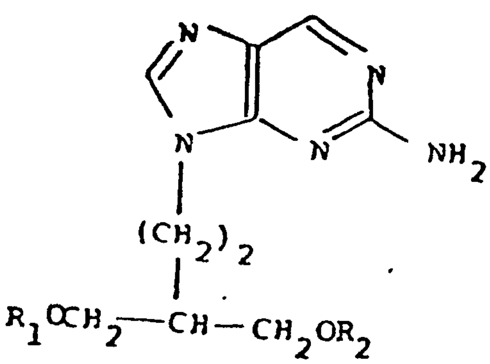

(I)

or a salt thereof, wherein $R_1$ and $R_2$ are each independently hydrogen, acyl or phosphate, provided that when one of $R_1$ or $R_2$ is phosphate, the other is hydrogen; or $R_1$ and $R_2$ are joined together to form a cyclic acetal group, a cyclic carbonate group or a cyclic phosphate group; and wherein the acyl group $R_1$ and/or $R_2$ is a group

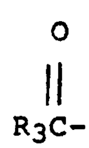

wherein $R_3$ is $C_{1-6}$ alkyl, $C_{1-6}$ alkoxy or optionally substituted aryl.

2. A compound according to claim 1 wherein $R_1$ and $R_2$ are hydrogen, or $R_1$ and/or $R_2$ is an acyl group

such that the group $R_1O$- and/or $R_2O$- is a pharmaceutically acceptable ester group.

3. A compound according to claim 1 wherein $R_1$ and $R_2$ are joined together to form a group

$$>C{=}O,\ >P(O)OH \text{ or}$$

$$>C(C_{1-3}\text{alkyl})_2, \text{ such as } >C(CH_3)_2,$$

or one of $R_1$ or $R_2$ is phosphate and the other is hydrogen.

4. A compound according to claim 1 selected from
   2-amino-9-(4-hydroxy-3-hydroxymethylbut-1-yl)purine;
   2-amino-9-(4-acetoxy-3-hydroxymethylbut-1-yl)purine;
   2-amino-9-(3-hydroxymethyl-4-methoxycarbonyloxybut -1-yl)purine;
   2-amino-9-[2-(2,2-dimethyl-1,3-dioxan-5-yl)ethyl] purine;
   2-amino-9-(4-propionyloxy-3-propionyloxymethylbut-1-yl) purine;
   2-amino-9-(4-butyryloxy-3-hydroxymethylbut-1-yl)purine;
   2-amino-9-(4-benzoyloxy-3-hydroxymethylbut-1-yl)purine;
   2-amino-9-(4-hydroxy-3-hydroxymethylbut-1-yl)purine 4'-phosphate;
   2-amino-9-(4-hydroxy-3-hydroxymethylbut-1-yl)purine 4': 4' 'phosphate;
   and pharmaceutically acceptable salts thereof.

5. 2-Amino-9-(4-acetoxy-3-acetoxymethylbut-1-yl)purine or a pharmaceutically acceptable salt thereof.

6. A process for preparing a compound of formula (I) as defined in claim 1 which process comprises either
   (a) where $R_1$ and $R_2$ are hydrogen, hydrolysing a compound of formula (II)

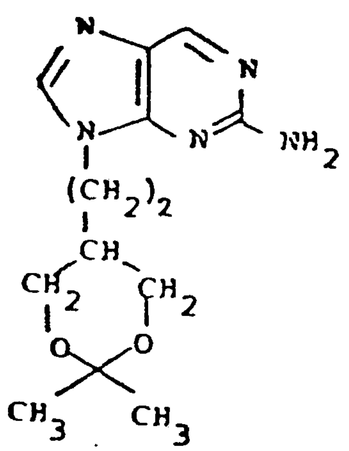

(II)

   (b) where $R_1$ and $R_2$ are acyl or joined together to form a cyclic carbonate, reducing a compound of formula (VIII)

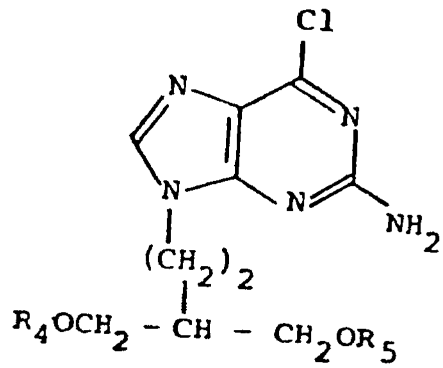

(VIII)

wherein $R_4$ and $R_5$ are the same or different acyl groups or $R_4$ and $R_5$ are joined together to form a cyclic carbonate group; or
(c) where one of $R_1$ or $R_2$ is phosphate or $R_1$ and $R_2$ together form a cyclic phosphate, by phosphorylating a compound of formula (XIII)

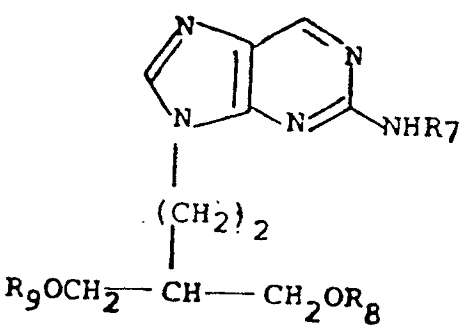

(XIII)

where $R_7$ is a protecting group and $R_8$ and $R_9$ are hydrogen or a protecting group provided that one of $R_8$ or $R_9$ is hydrogen, and thereafter if necessary deprotecting the product; and thereafter if desired carrying out one or more of the following steps:
i) converting a group $R_1$ and/or $R_2$ to another such group by acylation/deacylation, hydrolysis, acetalation or phosphate/carbonate formation;
ii) where the product is a salt forming a free base or a different salt thereof;
iii) where the product is a free base, forming an acid addition salt thereof; and
iv) where the product contains a phosphate group, forming a salt thereof.

7. A pharmaceutical composition comprising a compound of formula (I) as defined in claim 1 in combination with a pharmaceutically acceptable carrier.

8. A compound of formula (I) as defined in any one of claims 1 to 5. for use as an active therapeutic substance.

9. A compound of formula (I) as defined in any one of claims 1 to 5 for use in the treatment of viral infections in human or non-human animals.

10. The use of a compound of formula (I) as defined in any one of claims 1 to 5, in the manufacture of a medicament for use in the treatment of viral infections.

**Revendications**

1. Composé de formule (I) :

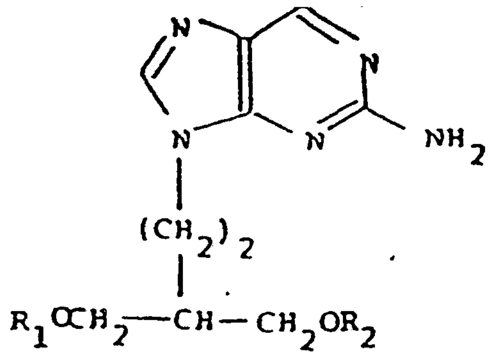

(I)

ou un sel de celui-ci, dans laquelle $R_1$ et $R_2$ sont chacun indépendamment un atome d'hydrogène, un groupe acyle ou phosphate, à condition que dans le cas où l'un des $R_1$ et $R_2$ est un groupe phosphate, l'autre est un atome d'hydrogène ; où $R_1$ et $R_2$ sont réunis pour fournir un groupe acétal cyclique, un groupe carbonate cyclique ou un groupe phosphate cyclique et où le groupe acyle $R_1$ et/ou $R_2$ est un groupe

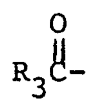 où $R_3$

est un groupe alkyle en $C_{1-4}$, alcoxy en $C_{1-6}$ ou aryle éventuellement substitué.

**2.** Composé suivant la revendication 1, caractérisé en ce que $R_1$ et $R_2$ sont un atome d'hydrogène, ou $R_1$ et/ou $R_2$ est un groupe acyle tel que le groupe $R_1O$ et/ou $R_2O$ est un groupe ester acceptable du point de vue pharmaceutique.

**3.** Composé suivant la revendication 1, caractérisé en ce que $R_1$ et $R_2$ sont réunis pour former un groupe

$$>C=O, \quad >P(O)OH \text{ ou } >C(\text{alkyle } C_{1-3})_2 \text{ , comme } >C(CH_3)_2 \text{ ,}$$

ou l'un des $R_1$ et $R_2$ est un groupe phosphate et l'autre est un atome d'hydrogène.

**4.** Composé suivant la revendication 1, caractérisé en ce qu'il est choisi parmi :
2-amino-9-(4-hydroxy-3-hydroxyméthylbut-1-yl)purine ;
2-amino-9-(4-acétoxy-3-hydroxyméthylbut-1-yl)purine ;
2-amino-9-(3-hydroxyméthyl-4-méthoxycarbonyloxybut-1-yl)purine ;
2-amino-9-[2-(2,2-diméthyl-1,3-dioxan-5-yl)éthyl]purine ;
2-amino-9-(4-propionyloxy-3-propionyloxyméthylbut-1-yl)purine ;
2-amino-9-(4-butyryloxy-3-hydroxyméthylbut-1-yl)purine ;
2-amino-9-(4-benzoyloxy-3-hydroxyméthylbut-1-yl)purine ;
4'-phosphate de 2-amino-9-(4-hydroxy-3-hydroxyméthylbut-1-yl)purine ;
4':4"-phosphate de 2-amino-9-(4-hydroxy-3-hydroxyméthylbut-1-yl)purine ;
et leurs sels acceptables du point de vue pharmaceutique.

**5.** Composé caractérisé en ce qu'il s'agit de la 2-amino-9-(4-acétoxy-3-acétoxyméthylbut-1-yl)purine ou d'un sel de celle-ci accpetable du point de vue pharmaceutique.

**6.** Procédé pour préparer un composé de formule (I) suivant la revendication 1, caractérisé en ce qu'il comprend :
(a) lorsque $R_1$ et $R_2$ sont un atome d'hydrogène, l'hydrolyse d'un composé de formule (II) :

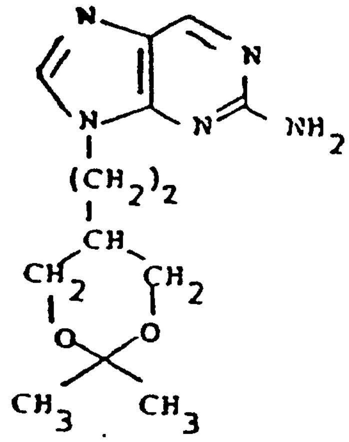

(II)

(b) lorsque $R_1$ et $R_2$ sont un groupe acyle ou réunis pour former un carbonate cyclique, la réduction d'un composé de formule (VIII) :

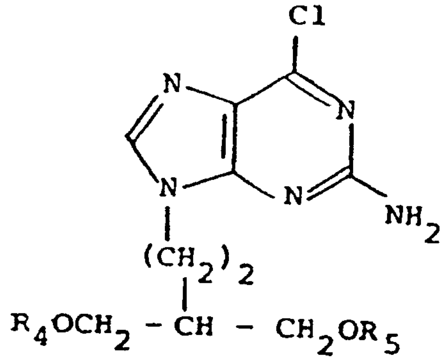

(VIII)

dans laquelle $R_4$ et $R_5$ sont des groupes acyles identiques ou différents ou $R_4$ et $R_5$ sont réunis pour former un groupe carbonate cyclique ; ou
(c) lorsque l'un des groupes $R_1$ ou $R_2$ est un groupe phosphate ou $R_1$ et $R_2$ forment ensemble un phosphate cyclique, la phosphorylation d'un composé de formule (XIII) :

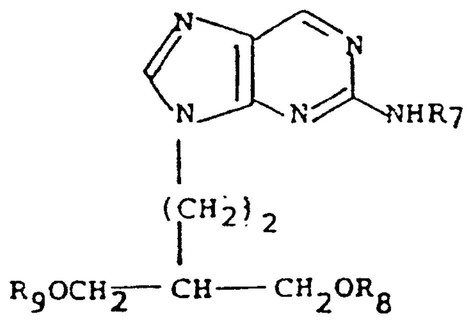

(XIII)

dans laquelle $R_7$ est un groupe protecteur et $R_8$ et $R_9$ sont un atome d'hydrogène ou un groupe protecteur à condition que l'un des $R_8$ et $R_9$ soit un atome d'hydrogène, et ensuite, si nécessaire, la déprotection du produit ; et ensuite, si cela est désiré, la réalisation d'une ou de plusieurs des étapes suivantes :
(i) conversion d'un groupe $R_1$ et/ou $R_2$ en un autre groupe analogue par acylation/désacylation, hydrolyse, acétalation ou formation de phosphate/carbonate ;
(ii) lorsque le produit est un sel, la formation d'une base libre ou d'un sel différent de celle-ci ;
(iii) lorsque le produit est une base libre, la formation d'un sel d'addition acide de celui-ci ; et
(iv) lorsque le produit contient un groupe phosphate, la formation d'un sel de celui-ci.

7. Composition pharmaceutique, caractérisée en ce qu'elle comprend un composé de formule (I) suivant la revendication 1, en association avec un support acceptable du point de vue pharmaceutique.

8. Composé de formule (I) suivant l'une quelconque des revendications 1 à 5, utile en tant que substance thérapeutique active.

9. Composé de formule (I) suivant l'une quelconque des revendications 1 à 5, utile dans le traitement d'infections virales chez des hommes ou des animaux non-humains.

10. Utilisation d'un composé de formule (I) suivant l'une quelconque des revendications 1 à 5, dans la fabrication d'un médicament utile dans le traitement d'infections virales.

## Ansprüche

1. Verbindung der Formel (I)

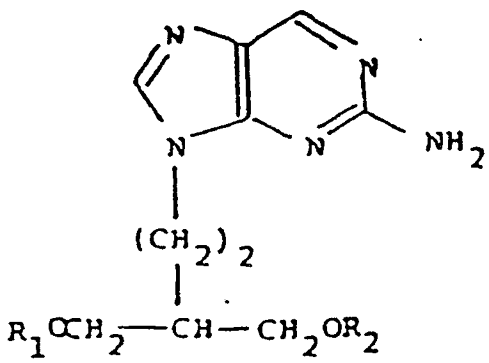

(I)

oder ein Salz davon, in der $R_1$ und $R_2$ jeweils unabhängig voneinander Wasserstoffatome, Acylreste oder Phosphatgruppen bedeuten, mit der Maßgabe, daß wenn eine der Gruppen $R_1$ oder $R_2$ eine Phosphatgruppe bedeutet, die andere ein Wasserstoffatom ist; oder die Reste $R_1$ und $R_2$ zusammengenommen einen cyclischen Acetalrest, einen cyclischen Carbonatrest oder einen cyclischen Phosphatrest darstellen; und in der der Acylrest $R_1$ und/oder $R_2$ einen Rest der Formel

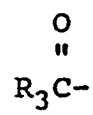

bedeutet, in der $R_3$ einen $C_{1-6}$-Alkyl-, $C_{1-6}$-Alkoxy- oder gegebenenfalls substituierten Arylrest darstellt.

2. Verbindung nach Anspruch 1, in der $R_1$ und $R_2$ Wasserstoffatome oder $R_1$ und/oder $R_2$ einen Acylrest bedeuten, so daß die Reste $R_1O$- und/oder $R_2O$- einen pharmazeutisch verträglichen Esterrest darstellen.

3. Verbindung nach Anspruch 1, in der $R_1$ und $R_2$ zusammengenommen einen Rest >C=O, >P(O)OH oder >C($C_{1-3}$alkyl)$_2$, wie >C($CH_3$)$_2$, darstellen oder einer der Reste $R_1$ oder $R_2$ einen Phosphatrest und der andere ein Wasserstoffatom bedeutet.

4. Verbindung nach Anspruch 1, nämlich
   2-Amino-9-(4-hydroxy-3-hydroxymethylbut-1-yl)-purin;
   2-Amino-9-(4-acetoxy-3-hydroxymethylbut-1-yl)-purin;
   2-Amino-9-(3-hydroxymethyl-4-methoxycarbonyloxybut-1-yl)-purin;
   2-Amino-9-[2-(2,2-dimethyl-1,3-dioxan-5-yl)-ethyl]-purin;
   2-Amino-9-(4-propionyloxy-3-propionyloxymethylbut-1-yl)-purin;
   2-Amino-9-(4-butyryloxy-3-hydroxymethylbut-1-yl)-purin;
   2-Amino-9-(4-benzoyloxy-3-hydroxymethylbut-1-yl)-purin;

2-Amino-9-(4-hydroxy-3-hydroxymethylbut-1-yl)-purin-4'-phosphat oder
2-Amino-9-(4-hydroxy-3-hydroxymethylbut-1-yl)-purin-4':4"-phosphat
und pharmazeutisch verträgliche Salze davon.

**5.** 2-Amino-9-(4-acetoxy-3-acetoxymethylbut-1-yl)-purin oder ein pharmazeutisch verträgliches Salz davon.

**6.** Verfahren zur Herstellung einer Verbindung der Formel (I) nach Anspruch 1, wobei das Verfahren umfaßt, entweder

(a) falls $R_1$ und $R_2$ Wasserstoffatome bedeuten, Hydrolysieren einer Verbindung der Formel (II)

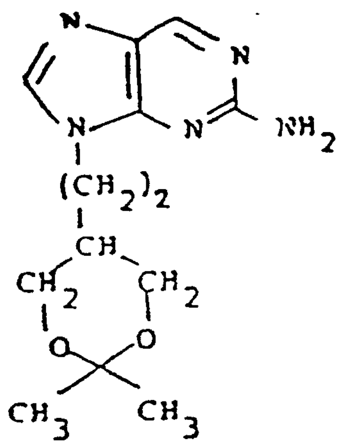

(II)

(b) falls $R_1$ und $R_2$ einen Acylrest oder zusammengenommen einen cyclischen Carbonatrest darstellen, Reduzieren einer Verbindung der Formel (VIII)

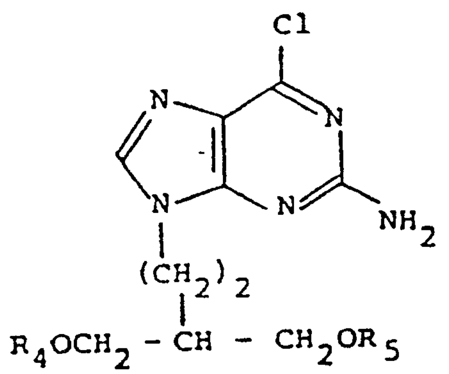

(VIII)

in der $R_4$ und $R_5$ die gleichen oder verschiedene Acylreste oder $R_4$ und $R_5$ zusammengenommen einen cyclischen Carbonatrest darstellen; oder

(c) falls einer der Reste $R_1$ oder $R_2$ einen Phosphatrest darstellt oder $R_1$ und $R_2$ zusammengenommen einen cyclischen Phosphatrest bedeuten, Phosphorylieren einer Verbindung der allgemeinen Formel (XIII)

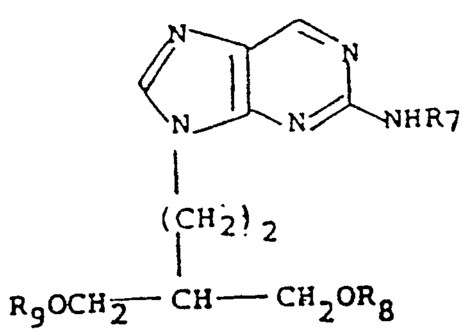

(XIII)

wobei $R_7$ eine Schutzgruppe bedeutet und $R_8$ und $R_9$ Wasserstoffatome oder eine Schutzgruppe darstellen, mit der Maßgabe, daß einer der Reste $R_8$ oder $R_9$ ein Wasserstoffatom bedeutet und, falls erforderlich, anschließend Entfernung der Schutzgruppen aus dem Produkt; und danach, falls erwünscht, Durchführung eines oder mehrerer der nachstehenden Schritte:
i) Umwandlung eines Restes $R_1$ und/oder $R_2$ in einen anderen derartigen Rest durch Acylierung/Entacylierung, Hydrolyse, Acetalysierung oder Herstellung eines Phosphates/Carbonates;
ii) falls das Produkt ein Salz ist, Herstellen einer freien Base oder eines anderen Salzes davon;
iii) falls das Produkt eine freie Base ist, Herstellung eines Säureadditionssalzes davon und
iv) falls das Produkt einen Phosphatrest enthält, Herstellung eines Salzes davon.

**7.** Arzneimittel, umfassend eine Verbindung der Formel (I) nach Anspruch 1 in Verbindung mit einem pharmazeutisch verträglichen Träger.

**8.** Verbindung der Formel (I) nach einem der Ansprüche 1 bis 5 zur Verwendung als einen therapeutisch wirksamen Stoff.

**9.** Verbindung der Formel (I) nach einem der Ansprüche 1 bis 5 zur Verwendung bei der Behandlung von Virusinfektionen von Menschen oder Tieren.

**10.** Verwendung einer Verbindung der Formel (I) nach einem der Ansprüche 1 bis 5 zur Herstellung eines Arzneimittels zur Verwendung bei der Behandlung von Virusinfektionen.